Europäisches Patentamt

European Patent Office

Office européen des brevets

(11) Numéro de publication : **0 291 421 B1**

(12) **FASCICULE DE BREVET EUROPEEN**

(45) Date de publication du fascicule du brevet :
27.03.91 Bulletin 91/13

(51) Int. Cl.⁵ : **A61M 1/16**

(21) Numéro de dépôt : 88420125.2

(22) Date de dépôt : 20.04.88

(54) Procédé de détermination de la natrémie d'un patient et rein artificiel en faisant application.

(30) Priorité : 15.05.87 FR 8707017

(43) Date de publication de la demande :
17.11.88 Bulletin 88/46

(45) Mention de la délivrance du brevet :
27.03.91 Bulletin 91/13

(84) Etats contractants désignés :
BE CH DE ES FR GB IT LI NL SE

(56) Documents cités :
DE-A- 3 524 823
US-A- 4 197 196
US-A- 4 601 830

(73) Titulaire : **HOSPAL INDUSTRIE**
**7, Avenue Lionel Terray**
**F-69330 Meyzieu (FR)**

(72) Inventeur : **Chevallet, Jacques**
**8, route de Ternay**
**F-69360 Serezin du Rahone (FR)**

## Description

La présente invention entre dans le domaine technique de l'analyse du sang circulant dans un appareil qui assure son épuration et que l'on appelle communément rein artificiel.

Plus particulièrement, la présente invention concerne un procédé permettant de déterminer la concentration en sodium du sang, ainsi qu'un rein artificiel comportant des moyens pour assurer la mise en oeuvre de ce procédé.

L'épuration du sang au moyen d'un rein artificiel met en oeuvre différents modes d'échanges entre le sang et un liquide d'épuration appelé liquide de dialyse. Ces échanges ont lieu à travers une membrane semi-perméable permettant l'épuration du sang à la fois par dialyse et par ultrafiltration.

Le phénomème de dialyse ou de diffusion est une conséquence de la différence de concentration en solutés existant entre le sang et le liquide de dialyse. Les substances présentes dans le sang à une concentration plus importante que dans le liquide de dialyse ont tendance à traverser la membrane de l'échangeur jusqu'à établir l'équilibre des concentrations. Ainsi, si l'on veut éliminer certaines substances en excès dans le sang, on fait circuler de l'autre côté de la membrane un liquide de dialyse qui en est dépourvu. A l'inverse, si on souhaite enrichir le sang en une substance donnée, on choisit un liquide de dialyse dont la concentration en cette substance est plus importante que celle du sang.

Le second mode d'épuration utilisé dans un rein artificiel est l'ultrafiltration qui est la conséquence de la différence de pression existant entre les deux liquides de part et d'autre de la membrane. Dans le cas où la pression du sang est supérieure à la pression du liquide de dialyse, une partie de la fraction aqueuse du sang traverse la membrane semi-perméable et est éliminée ensuite avec le liquide de dialyse.

Ce phénomène d'ultrafiltration permet notamment l'élimination de la surcharge hydrique du patient. En effet, chez un patient hémodialysé chronique, l'eau qui devrait normalement être éliminée par les reins, s'accumule dans l'organisme entre deux séances d'hémodialyse. Cet excédent hydrique se répartit entre le compartiment intracellulaire et le compartiment extracellulaire dont notamment le système vasculaire.

Bien que, durant la séance d'hémodialyse, le seul compartiment directement accessible soit le milieu vasculaire, il n'est pas possible d'éliminer totalement la surcharge hydrique du patient par simple ultrafiltration du sang. On risquerait en effet, très vite, de provoquer chez le patient des chutes de tension dûes à la diminution du volume sanguin. D'autre part, si l'on se contente de corriger l'hyperhydratation extracellulaire, on provoque chez le patient hémodialysé le syndrôme bien connu de déséquilibre qui se traduit notamment par des maux de tête, nausées, crampes.

Il importe donc, au cours de la séance d'hémodialyse, non seulement d'atteindre la perte de poids désirée, mais aussi de contrôler l'équilibre hydrique entre les différents compartiments de l'organisme.

L'un des facteurs permettant de contrôler cet équilibre hydrique est la natrémie du patient. Le médecin qui surveille le déroulement de la séance d'hémodialyse a donc besoin de surveiller la natrémie du patient pour modifier ensuite, si cela est nécessaire, les conditions de traitement du sang.

Pour connaître la valeur de la natrémie d'un patient, il est connu selon l'art antérieur de prélever des échantillons de sang veineux et de les analyser ensuite, au moyen d'une électrode spécifique au sodium par exemple.

Cette méthode, bien que fiable, présente cependant l'inconvénient d'être lourde et onéreuse, du fait notamment des nombreux étalonnages nécessaires. Il n'est d'autre part, pas conseillé d'effectuer de trop nombreux prélèvements sanguins chez un patient hémodialysé qui, en général, souffre déjà d'anémie.

Pour pallier à ces inconvénients, le Dr. Petitclerc propose, dans sa thèse du 13 juin 1985, de substituer à la mesure de la natrémie une mesure de la conductivité du liquide de dialyse en équilibre avec le plasma sanguin. En effet, il démontre dans cette thèse, la très bonne corrélation existant entre ces deux valeurs.

Pour réaliser l'équilibre entre le plasma et le liquide de dialyse, le Dr. Petitclerc propose de faire recirculer une petite quantité de liquide de dialyse dans l'échangeur, jusqu'à obtention ou presque de l'équilibre. Cette phase de recirculation dure environ 10 minutes et doit être effectuée, à chaque nouvelle mesure. Durant cette phase de recirculation, l'ultrafiltration est maintenue.

Ce procédé d'obtention de l'équilibre entre le liquide de dialyse et le plasma sanguin présente un très grave inconvénient. En effet, à chaque phase de mesure, l'épuration du sang par dialyse est très vite limitée du fait de la recirculation du liquide de dialyse. Ainsi, dans le cas où on souhaite effectuer des déterminations fréquentes de la natrémie, l'épuration du sang par dialyse devient insuffisante si on ne prolonge pas la durée de la séance d'hémodialyse.

Pour permettre une bonne épuration du sang par dialyse, il faut donc ou prolonger la durée du traitement du sang, ou limiter le nombre de mesures effectuées. Quelle que soit l'alternative choisie, la solution n'est nullement satisfaisante.

L'objet de la présente invention est donc de pallier aux inconvénients de l'art antérieur et de proposer un procédé et un rein artificiel permettant de déterminer la natrémie d'un patient d'une manière simple, rapide, fiable et peu coûteuse.

Un autre objet de la présente invention est de proposer un procédé et un rein artificiel permettant de déterminer la natrémie d'un patient sans avoir à prélever d'échantillon de sang veineux.

Un autre objet de la présente invention est de proposer un procédé et un rein artificiel permettant de déterminer la natrémie d'un patient sans affecter l'épuration du sang.

Un autre objet de la présente invention est de proposer un procédé et un rein artificiel permettant de déterminer la natrémie d'un patient aussi fréquemment qu'on le souhaite.

Pour réaliser les objectifs cités ci-dessus, la présente invention a pour objet un procédé de détermination de la natrémie d'un patient dont le sang circule dans le premier compartiment d'un échangeur séparé par une membrane semi-perméable permettant la dialyse et l'ultrafiltration du sang d'un second compartiment traversé par un circuit de liquide de dialyse, selon lequel on détermine la conductivité du liquide de dialyse en équilibre avec le plasma puis on effectue la correspondance entre cette valeur de la conductivité et la concentration en sodium du plasma. Ce procédé est caractérisé en ce qu'on détermine la conductivité du liquide de dialyse en équilibre avec le plasma en effectuant les opérations suivantes :

    – on fait varier, de façon déterminée, la conductivité du liquide de dialyse à l'entrée de l'échangeur,

    – on mesure la conductivité du liquide de dialyse à la sortie de l'échangeur,

    – on détermine le temps de retard Tr du liquide de dialyse entre l'entrée et la sortie de l'échangeur,

    – on détermine la valeur de la conductivité pour laquelle la conductivité du liquide de dialyse à la sortie de l'échangeur à un instant t est égale, à la conductivité du liquide de dialyse à l'entrée de l'échangeur à un instant $t' = t - Tr$, cette valeur étant la valeur de la conductivité du liquide de dialyse en équilibre avec le plasma.

La présente invention a également pour objet un rein artificiel comprenant un échangeur à au moins deux compartiments séparés par une membrane semi-perméable permettant la dialyse et l'ultrafiltration du sang, le premier compartiment étant relié à un circuit extracorporel de sang, le second compartiment étant traversé par un circuit de liquide de dialyse comportant des moyens pour faire varier de façon déterminée la conductivité du liquide de dialyse à l'entrée de l'échangeur, des moyens pour mesurer la conductivité du liquide de dialyse à la sortie de l'échangeur, caractérisé en ce que ledit circuit de liquide de dialyse comporte en outre :

    – des moyens pour déterminer le temps de retard Tr du liquide de dialyse entre l'entrée et la sortie de l'échangeur,

    – des moyens pour déterminer la valeur de conductivité du liquide de dialyse en équilibre avec le plasma pour laquelle la conductivité du liquide de dialyse à la sortie de l'échangeur à un instant t est égale, à la conductivité du liquide de dialyse à l'entrée de l'échangeur à un instant $t' = t - Tr$.

D'autres objets et avantages de la présente invention apparaîtront au cours de la description qui va suivre, en référence aux figures annexées qui illustrent schématiquement et sans échelle déterminée, des modes de réalisation de la présente invention.

La figure I représente, de façon schématique, un mode de réalisation du rein artificiel objet de la présente invention.

La figure II est une représentation graphique d'un mode de réalisation du procédé objet de la présente invention.

La figure III est une représentation graphique de la détermination du temps de retard Tr selon un mode de réalisation de la présente invention.

La figure IV est une représentation sous forme de schéma blocs du dispositif de contrôle 9 de la présente invention.

Les figures V et VI illustrent des variantes de réalisation de la présente invention.

En se référant à la figure I, on voit que le rein artificiel selon la présente invention comporte un échangeur (1) de tout type connu comprenant au moins deux compartiments (3, 4) séparés par une membrane semi-perméable (2). Le premier compartiment (3) est relié à un patient (non représenté) par un circuit extracorporel de sang (5), alors que le second compartiment (4) est traversé par un circuit de liquide de dialyse (6).

Ce circuit de liquide de dialyse (6) comporte en amont de l'échangeur (1), de façon conventionnelle, un dispositif (10) de préparation du liquide de dialyse qui est relié à une source de concentré ainsi qu'à une alimentation d'eau (non représentées). La conductivité du liquide de dialyse à l'entrée de l'échangeur peut être contrôlée grâce au conductimètre (7).

Un second conductimètre (8) mesure la conductivité du liquide de dialyse à la sortie de l'échangeur (1), avant qu'il soit conduit à des moyens d'évacuation ou de recyclage (non représentés).

Le circuit de liquide de dialyse (6) comporte en outre, bien que, pour des raisons de clarté, ils ne soient pas représentés, tous les éléments nécessaires à une circulation correcte du liquide de dialyse : pompes, débitmètres, dispositifs de dégazage, détecteur de sang, vannes... etc, qui ne sont pas critiques au regard de la présente invention.

Les conductimètres (7, 8) sont reliés à un dispositif de contrôle (9) qui est relié également à la cuve de préparation (10) du liquide de dialyse.

Le procédé de détermination de la conductivité du liquide de dialyse en équilibre avec le plasma est illustré sur la figure II. Sur cette figure sont représentées l'évolution en fonction du temps de la conductivité du liquide de dialyse à l'entrée de l'échangeur (courbe E) ainsi que l'évolution en fonction du temps de la conductivité du liquide de dialyse à la sortie de l'échangeur (courbe S). L'espace de temps représenté ici est l'espace de temps correspondant à la mesure.

Les valeurs de la courbe S sont les valeurs mesurées grâce au conductimètre 8. Les valeurs de la courbe E sont, soit les valeurs de conductivité mesurées par le conductimètre 7, soit les valeurs de conductivité fixées par avance par le dispositif de contrôle 9 ou par tout autre dispositif permettant d'imposer la conductivité du liquide de dialyse préparé par le dispositif 10.

La détermination de la conductivité du liquide de dialyse en équilibre avec le plasma sera cependant plus précise si les valeurs retenues pour le tracé de la courbe E sont les valeurs effectivement mesurées à l'entrée de l'échangeur par le conductimètre 7, ce qui est le cas choisi pour la description qui va suivre.

Ainsi, au moment où l'on souhaite effectuer une mesure, on impose une variation déterminée de la conductivité du liquide de dialyse à l'entrée de l'échangeur, grâce à une action du dispositif de contrôle 9 sur le dispositif de préparation 10. Ceci est possible grâce à un générateur de liquide de dialyse capable de fournir un liquide dont la concentration en sodium est variable.

Cette action a pour conséquence une variation de la conductivité du liquide de dialyse mesurée par le conductimètre 7, à partir de l'instant t0, ce qui se traduit sur la courbe E par un changement de pente.

L'observation de la courbe S montre que si la conductivité à l'entrée de l'échangeur commence à varier à partir de l'instant t0, la conductivité du liquide de dialyse à la sortie de l'échangeur qui est mesurée par le conductimètre 8 ne commence à varier qu'à partir de l'instant t1. L'intervalle de temps séparant l'instant t0 de l'instant t1 est le temps de retard Tr qui est le délai nécessaire pour qu'une variation de conductivité mesurée par le conductimètre 7 entraîne une variation de la conductivité que l'on mesure par le conductimètre 8. Ce temps de retard Tr est déterminé selon un procédé qui sera expliqué plus bas.

Le dispositif de contrôle 9 détermine la conductivité du liquide de dialyse en équilibre avec le plasma en comparant, pour chaque valeur de la conductivité du liquide de dialyse mesurée à la sortie de l'échangeur 1 par le conductimètre 8 à un instant t, la valeur de la conductivité correspondante à l'entrée de l'échangeur, c'est à dire la valeur mesurée par le conductimètre 7 à l'instant t' = t − Tr.

La conductivité pour laquelle ces deux valeurs sont égales est la conductivité du liquide de dialyse en équilibre avec le plasma.

Ainsi, sur la figure II, la conductivité du liquide de dialyse à l'équilibre $\gamma$ eq est la conductivité mesurée par le conductimètre 8 à l'instant t3 qui est égale à la conductivité mesurée par le conductimètre 7 à l'instant t2 = t3 − Tr.

En effet, étant donné que la conductivité du liquide de dialyse varie essentiellement en fonction de sa concentration en sodium, on estime que si la conductivité du liquide de dialyse ne varie pas lors de son passage dans l'échangeur, c'est que la concentration en sodium ne varie pas non plus. On considère alors que l'équilibre est réalisé entre le liquide de dialyse circulant d'un côté de la membrane et le plasma du sang circulant de l'autre côté de la membrane.

A partir de cette valeur de la conductivité du liquide de dialyse à l'équilibre, on peut ensuite déterminer la natrémie du patient. La correspondance entre ces deux valeurs peut s'effectuer en deux étapes : tout d'abord conversion de la conductivité du liquide de dialyse en concentration sodée, puis en natrémie.

La très bonne corrélation existant entre la concentration en sodium du liquide de dialyse et la mesure de conductivité est illustrée au dernier chapitre de la thèse déjà citée du Dr. Petitclerc. En effet, le sodium représente avec l'anion qui l'accompagne nécessairement pour assurer la neutralité électrique, 95% environ de l'osmolalité ionique du liquide de dialyse. Ainsi, à condition de tenir compte des variations éventuelles de température, on peut suivre les variations en sodium du liquide de dialyse grâce aux mesures de la conductivité.

La seconde étape effectuée est la détermination de la natrémie à partir de la concentration sodée du liquide de dialyse. Le sodium étant une particule électriquement chargée, le rapport des concentrations sodées de part et d'autre de la membrane est égal au coefficient de Donnan. Cette relation a été décrite par le Dr. Petitclerc dans un article intitulé "Clinical Validation of a Predictive Modeling Equation for Sodium" (Artificial Organs, vol. 9 No 2, 1985).

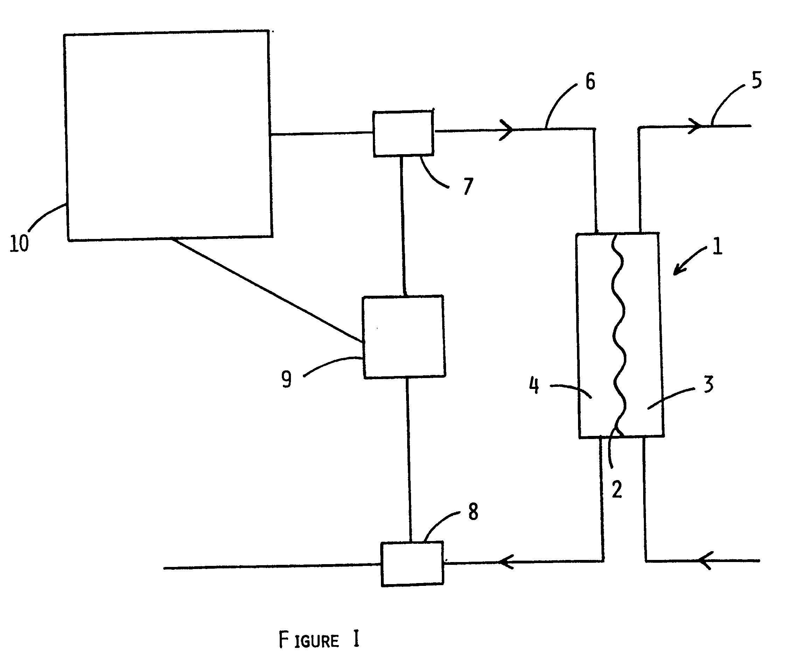

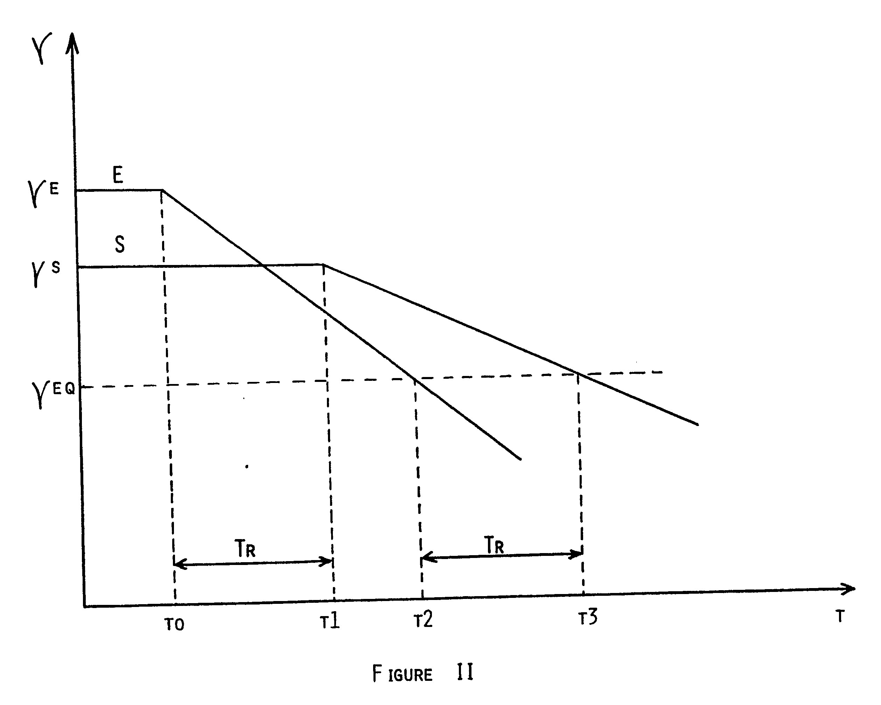

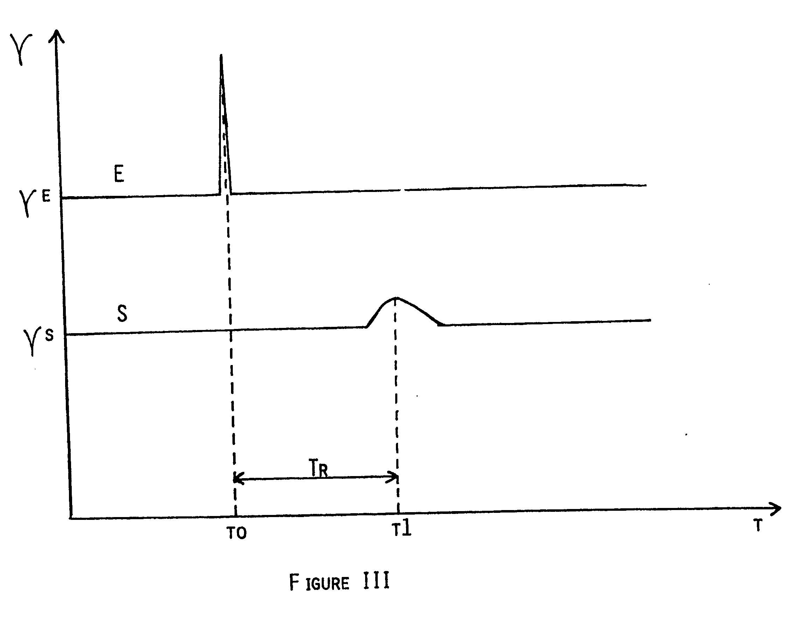

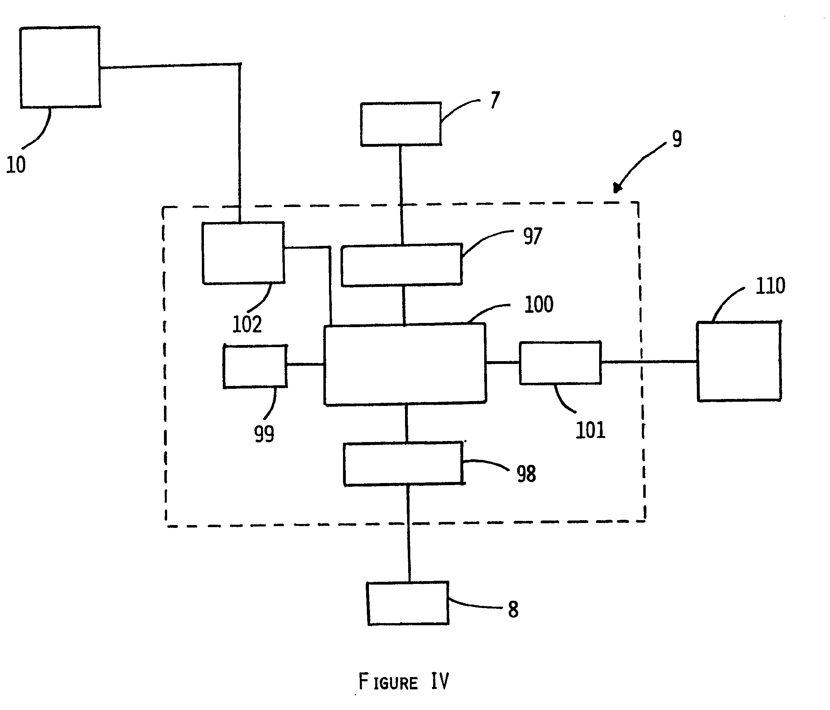

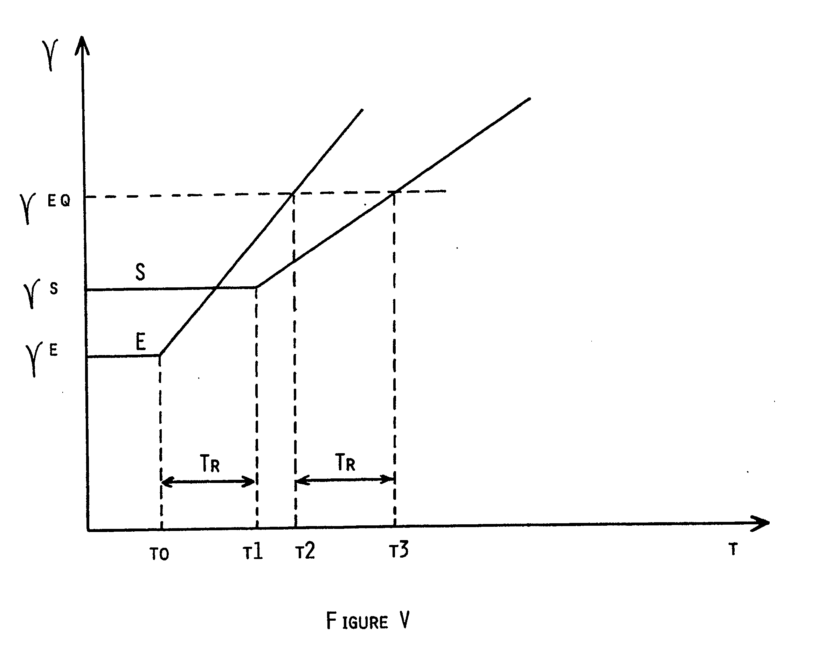

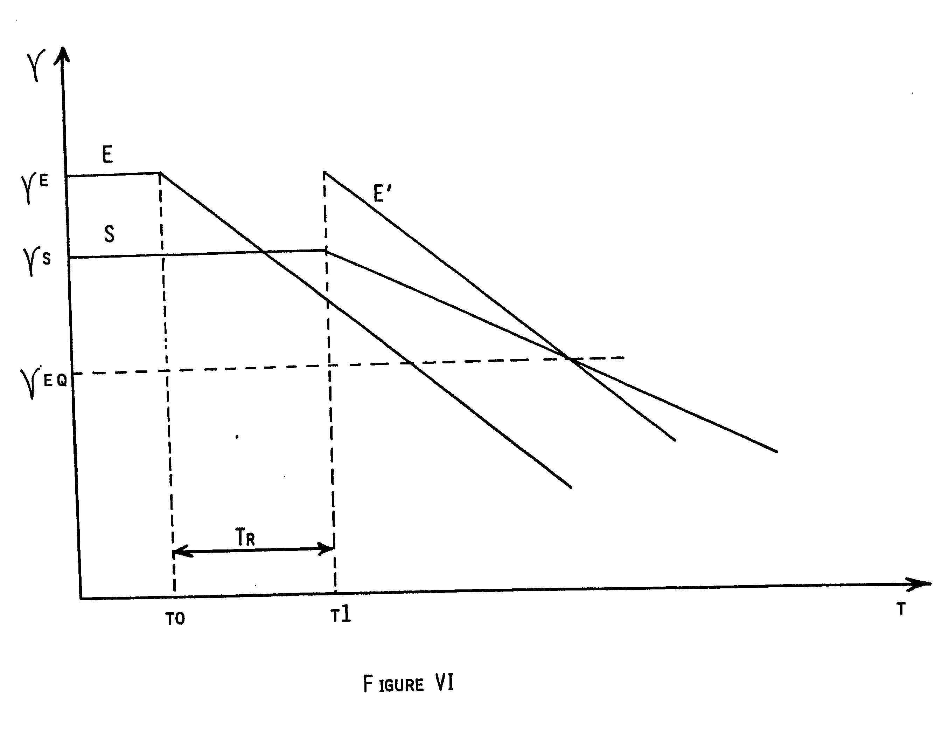

La relation entre la conductivité du liquide de dialyse en équilibre et la natrémie peut être établie directement en s'appuyant sur les résultats publiés dans le chapitre 6 (figure 5 et page 156) de la thèse précédemment citée du Dr. Petitclerc, montrant la bonne corrélation existant entre la conductivité du liquide de dialyse en équilibre et la concentration sodée efficace que l'on sait équivalente à la natrémie.

La figure III illustre la détermination selon un des modes de réalisation de la présente invention du temps de retard Tr qui est le délai nécessaire pour qu'une variation de la conductivité imposée à l'entrée de l'échangeur entraîne une variation de la conductivité mesurée par le conductimètre 8.

Si, pour déterminer la conductivité du liquide de dialyse en équilibre avec le plasma, on choisit comme valeur de conductivité à l'entrée de l'échangeur, la valeur mesurée par le conductimètre 7, alors le temps de retard Tr est le délai nécessaire pour qu'une variation de conductivité mesurée par le conductimètre 7 entraîne une variation de la valeur mesurée à la sortie de l'échangeur par le conductimètre 8. C'est le cas décrit ci-dessous.

Par contre, dans le cas non préférentiel, où on choisit comme valeur de la conductivité à l'entrée de l'échangeur les valeurs fixées par avance par le dispositif de contrôle, le temps de retard Tr est le délai séparant la consigne de variation de la conductivité par le dispositif de contrôle 9 et la variation de conductivité mesurée par le conductimètre 8.

La courbe E de la figure III, représente, de la même façon que sur la figure II, l'évolution dans le temps de la conductivité du liquide de dialyse à l'entrée de l'échangeur, alors que la courbe S représente l'évolution dans le temps de la conductivité du liquide de dialyse à la sortie de l'échangeur.

Pour effectuer cette détermination du temps de retard, on impose une variation importante mais brève de la conductivité du liquide de dialyse préparé dans le dispositif 10, ce qui se traduit sur la courbe E par un pic de conductivité que l'on détecte à l'instant t0 grâce au conductimètre 7.

On mesure ensuite le temps qui s'écoule depuis l'instant t0 jusqu'à l'instant t1 où on détecte, grâce au conductimètre 8, la variation de conductivité du liquide de dialyse à la sortie de l'échangeur, qui est la réponse à la variation de conductivité provoquée à l'entrée de l'échangeur.

La durée séparant l'instant t0 de l'instant t1 est égale au temps de retard Tr.

Pour permettre de réaliser les différentes étapes du procédé décrit ci-dessus, le rein artificiel objet de la présente invention comporte un dispositif de contrôle 9 dont les différentes fonctions sont illustrées sous forme d'un schéma blocs sur la figure IV. Les informations provenant des conductimètres 7 et 8 sont reçues par le dispositif de contrôle 9 grâce à des organes récepteurs respectivement 97 et 98. Ces organes récepteurs sont constitués par exemple par des mémoires, reliées au calculateur 100. A partir des informations stockées dans ces organes 97 et 98, le calculateur 100 détermine la valeur du temps de retard Tr qui est ensuite mis en mémoire en 99, et détermine également la valeur de conductivité du liquide de dialyse en équilibre avec le plasma qui est la valeur pour laquelle la conductivité du liquide de dialyse à la sortie de l'échangeur à un instant t est égale à la conductivité du liquide de dialyse à l'entrée de l'échangeur à un instant $t' = t - Tr$.

Cette valeur de la conductivité du liquide de dialyse en équilibre avec le plasma est ensuite transmise au convertisseur 101 qui détermine alors la valeur de la natrémie correspondante.

Pour être accessible au médecin surveillant la séance d'hémodialyse, on peut prévoir des moyens d'affichage (110) du résultat de la mesure.

Le dispositif de contrôle 9 comporte également un système de commande 102 de la variation de conductivité du liquide de dialyse. Ce système de commande 102 agit sur le système de préparation 10 et est relié avantageusement au calculateur 100.

De façon préférentielle, le dispositif de contrôle 9 est un microprocesseur.

Selon un autre mode de réalisation de la présente invention, tel qu'illustré à la figure V, on fait varier la conductivité du liquide de dialyse à l'entrée de l'échangeur, non pas de façon décroissante, mais de façon croissante.

La variation de conductivité a été représentée sur les figures II et V de façon continue.

Il est également possible, de faire varier la conductivité de façon discontinue, par paliers successifs.

Selon un autre mode de réalisation de la présente invention, la détermination du temps de retard Tr est effectuée à postériori, après que l'on ait commencé à mémoriser les valeurs de conductivité à la sortie de l'échangeur. En effet, on garde en mémoire les différentes valeurs mesurées grâce au conductimètre 8 et on détermine l'équation de la courbe S dès que le nombre de mesures est suffisant.

Etant donné que la conductivité du liquide de dialyse à la sortie de l'échangeur est constante jusqu'à l'instant t1, on peut déterminer t1 qui est l'abcisse du point où la pente de la courbe S change.

On détermine alors facilement t1 en calculant l'abcisse du point vérifiant l'équation de S et dont l'ordonnée est la valeur de la conductivité mesurée grâce au conductimètre 8 avant que la période de mesure ne commence.

Le temps de retard Tr est ensuite déterminé par différence entre t0 et t1.

Selon un autre mode de réalisation de la présente invention, tel qu'illustré à la figure VI, on détermine la conductivité du liquide de dialyse en équilibre en effectuant une translation dans le temps, de distance Tr, de la courbe E. On obtient alors la courbe E'. L'équation des courbes E' et S étant déterminée, la valeur de la conductivité du liquide de dialyse en équilibre est l'ordonnée du point d'intersection des courbes E' et S.

La présente invention n'est pas limitée aux modes de réalisation décrits car de nombreuses variantes de réalisation sont à la portée du technicien sans pour autant sortir de son cadre.

## Revendications

1. Procédé de détermination de la natrémie d'un patient dont le sang circule dans le premier compartiment (3) d'un échangeur (1) séparé par une membrane (2) semi-perméable permettant la dialyse et l'ultrafiltration du sang d'un second compartiment (4) traversé par un circuit (6) de liquide de dialyse, selon lequel on détermine la conductivité du liquide de dialyse en équilibre avec le plasma puis on effectue la correspondance entre cette valeur de la conductivité et la concentration en sodium du plasma, caractérisé en ce qu'on détermine la conductivité du liquide de dialyse en équilibre avec la plasma en effectuant les opérations suivantes :

    – on fait varier, de façon déterminée, la conductivité du liquide de dialyse à l'entrée de l'échangeur,

    – on détermine le temps de retard $Tr$ du liquide de dialyse entre l'entrée et la sortie de l'échangeur,

    – on détermine la valeur de la conductivité pour laquelle la conductivité du liquide de dialyse à la sortie de l'échangeur est égale à un instant $t$ à la conductivité du liquide de dialyse à l'entrée de l'échangeur à un instant $t' = t - Tr$, cette valeur étant la valeur de la conductivité du liquide de dialyse en équilibre avec le plasma.

2. Procédé selon la revendication 1, caractérisé en ce qu'on fait varier la conductivité du liquide de dialyse à l'entrée de l'échangeur de façon croissante.

3. Procédé selon la revendication 1, caractérisé en ce qu'on fait varier la conductivité du liquide de dialyse à l'entrée de l'échangeur de façon décroissante.

4. Procédé selon l'une des revendications précédentes, caractérisé en ce qu'on fait varier la conductivité du liquide de dialyse à l'entrée de l'échangeur de façon continue.

5. Procédé selon l'une des revendications précédentes, caractérisé en ce que la détermination du temps de retard $Tr$ est effectuée de la manière suivante :

    – on provoque à un instant $t0$ une variation brutale mais brève de la conductivité du liquide de dialyse à l'entrée de l'échangeur,

    – on mesure le temps s'écoulant à partir de l'instant $t0$ jusqu'à l'instant $t1$ où on détecte grâce à un conductimètre (8) une variation de la conductivité du liquide de dialyse à la sortie de l'échangeur qui est la réponse à la variation de conductivité provoquée à l'entrée de l'échangeur.

6. Procédé selon les revendications 1 à 4, caractérisé en ce que la détermination du temps de retard $Tr$ est effectuée de la manière suivante :

    – on provoque à partir d'un instant $t0$ une variation déterminée de la conductivité du liquide de dialyse à l'entrée de l'échangeur,

    – on mesure la valeur de la conductivité du liquide de dialyse à la sortie de l'échangeur,

    – on détermine l'équation de la courbe S de l'évolution de la conductivité du liquide de dialyse à la sortie de l'échangeur en fonction du temps,

    – on détermine l'instant $t1$ qui est l'abcisse du point de changement de pente de la courbe S,

    – on détermine le temps de retard $Tr$ qui est l'intervalle de temps séparant l'instant $t0$ de l'instant $t1$.

7. Procédé selon l'une des revendications précédentes, caractérisé en ce que la valeur considérée de conductivité du liquide de dialyse à l'entrée de l'échangeur est la valeur mesurée par un conductimètre (7) placé en amont de l'échangeur (1) par rapport à la circulation du liquide de dialyse.

8. Procédé selon les revendications 1 à 6, caractérisé en ce que la valeur considérée de conductivité du liquide de dialyse à l'entrée de l'échangeur (1) est une valeur de consigne imposée par un dispositif de contrôle (9).

9. Rein artificiel comprenant un échangeur (1) à au moins deux compartiments (3, 4) séparés par une membrane semi-perméable (2) permettant la dialyse et l'ultrafiltration du sang, le premier compartiment (3) étant relié à un circuit (5) extracorporel de sang, le second compartiment (4) étant traversé par un circuit (6) de liquide de dialyse comportant des moyens (102) pour faire varier la conductivité du liquide de dialyse à l'entrée de l'échangeur (1), des moyens (8) pour mesurer la conductivité du liquide de dialyse à la sortie de l'échangeur (1), caractérisé en ce que ledit circuit (6) de liquide de dialyse comporte en outre

    – des moyens pour déterminer le temps de retard $Tr$ du liquide de dialyse entre l'entrée et la sortie de l'échangeur (1)

    – des moyens pour déterminer la valeur de conductivité du liquide de dialyse en équilibre avec le plasma pour laquelle la conductivité du liquide de dialyse à la sortie de l'échangeur (1) est égale à un instant $t$ à la conductivité du liquide de dialyse à l'entrée de l'échangeur à un instant $t'=t - Tr$.

10. Rein artificiel selon la revendication 9, caractérisé en ce qu'il comporte en outre des moyens (7) pour contrôler la conductivité du liquide de dialyse à l'entrée de l'échangeur (1).

11. Rein artificiel selon la revendication 9 ou 10, caractérisé en ce qu'il comporte en outre des moyens (101) pour déterminer la valeur de la natrémie à partir de la valeur de la conductivité du liquide de dialyse en équilibre avec le plasma.

12. Rein artificiel selon les revendications 9 à 11, caractérisé en ce que lesdits moyens pour déterminer le temps de retard $Tr$ ainsi que lesdits moyens pour déterminer la conductivité du liquide de dialyse en équilibre avec le plasma et lesdits moyens pour déterminer la valeur de la natrémie à partir de ladite valeur de conductivité du liquide de dialyse à l'équilibre sont constitués par un microprocesseur.

## Ansprüche

1. Verfahren zur Bestimmung der Natriämie eines Patienten, dessen Blut in eine erste Kammer (3) eines Austauschers (1) strömt, die durch eine semipermeable Membran (2), die die Dialyse und die Ultrafiltration des Blutes ermöglicht, von einer zweiten Kammer (4) getrennt ist, die von einem Dialyseflüssigkeitskreislauf (6) durchflossen wird, an welchem man die Leitfähigkeit der Dialyseflüssigkeit im Gleichgewicht mit dem Plasma bestimmt, worauf man dem Wert der Leitfähigkeit die Natriumkonzentration des Plasmas zuordnet, dadurch gekennzeichnet, daß man die Leitfähigkeit der Dialyseflüssigkeit im Gleichgewicht mit dem Plasma bestimmt, indem man folgende Arbeitsvorgänge ausführt :

– man ändert in bestimmter Weise die Leitfähigkeit der Dialyseflüssigkeit am Eintritt in den Austauscher,

– man bestimmt die Verzögerungszeit Tr der Dialyseflüssigkeit zwischen dem Eintritt in den Austauscher und dem Austritt aus dem Austauscher,

– man bestimmt den Wert der Leitfähigkeit, für welchen die Leitfähigkeit der Dialyseflüssigkeit am Austritt aus dem Austauscher zu einem Zeitpunkt t gleich der Leitfähigkeit der Dialyseflüssigkeit am Eintritt in den Austauscher zu einem Zeitpunkt t' = t – Tr ist, wobei dieser Wert der Wert der Leitfähigkeit der Dialyseflüssigkeit im Gleichgewicht mit dem Plasma ist.

2. Verfahren nach Anspruch 1, dadurch gekennzeichnet, daß man die Leitfähigkeit der Dialyseflüssigkeit am Eintritt in den Austauscher mit aufsteigender Charakteristik ändert.

3. Verfahren nach Anspruch 1, dadurch gekennzeichnet, daß man die Leitfähigkeit der Dialyseflüssigkeit am Eintritt in den Austauscher mit fallender Charakteristik ändert.

4. Verfahren nach einem der vorhergehenden Ansprüche, dadurch gekennzeichnet, daß man die Leitfähigkeit der Dialyseflüssigkeit am Eintritt in den Austauscher in gleichbleibender Charakteristik ändert.

5. Verfahren nach einem der vorhergehenden Ansprüche, dadurch gekennzeichnet, daß die Bestimmung der Verzögerungszeit Tr in folgender Art und Weise ausgeführt wird :

– man ruft zu einem Zeitpunkt t0 eine starke, aber kurze Änderung der Leitfähigkeit der Dialyseflüssigkeit beim Eintreten in den Austauscher hervor,

– man mißt die Zeit, die vom Zeitpunkt t0 bis zum Zeitpunkt t1 verstreicht, wo man mittels eines Leitfähigkeitsmeßgeräts (8) eine Änderung der Leitfähigkeit der Dialyseflüssigkeit am Austritt aus dem Austauscher wahrnimmt, die der am Eintritt in den Austauscher hervorgerufene Änderung der Leitfähigkeit entspricht.

6. Verfahren nach den Ansprüchen 1 bis 4, dadurch gekennzeichnet, daß die Bestimmung der Verzögerungszeit Tr in der folgenden Art und Weise ausgeführt wird :

– man ruft ab einem Zeitpunkt t0 eine bestimmte Änderung der Leitfähigkeit der Dialyseflüssigkeit beim Eintreten in den Austauscher hervor,

– man mißt den Wert der Leitfähigkeit der Dialyseflüssigkeit beim Austreten aus dem Austauscher,

– man bestimmt die Gleichung der Kurve S der Entwicklung der Leitfähigkeit der Dialyseflüssigkeit beim Austreten aus dem Austauscher als Funktion der Zeit,

– man bestimmt den Zeitpunkt t1, der der Abszissenpunkt bei der Änderung der Steigung der Kurve S ist,

– man bestimmt die Verzögerungszeit Tr, die das Zeitintervall zwischen dem Zeitpunkt t0 und dem Zeitpunkt t1 darstellt.

7. Verfahren nach einem der vorhergehenden Ansprüche, dadurch gekennzeichnet, daß der betrachtete Wert der Leitfähigkeit der Dialyseflüssigkeit beim Eintreten in den Austauscher der durch ein Leitfähigkeitsmeßgerät (7) gemessene Wert ist, das stromaufwärts vom Austauscher (1) bezüglich des Dialyseflüssigkeitskreislaufes angeordnet ist.

8. Verfahren nach den Ansprüchen 1 bis 6, dadurch gekennzeichnet, daß der betrachtete Wert der Leitfähigkeit der Dialyseflüssigkeit beim Eintreten in den Austauscher (1) ein durch eine Steuervorrichtung (9) auferlegter Einstellwert ist.

9. Künstliche Niere umfassend einen Austauscher (1) mit mindestens zwei Kammern (3, 4), die durch eine semipermeable Membran (2) getrennt sind, die die Dialyse und die Ultrafiltration des Blutes ermöglicht, wobei die erste Kammer (3) mit einem Blutkreislauf (5) außerhalb des Körpers verbunden ist, und die zweite Kammer (4) von einem Dialyseflüssigkeitskreislauf (6) durchflossen wird, der mittel (102) zum Ändern der Leitfähigkeit der Dialyseflüssigkeit beim Eintreten in den Austauscher (1) und mittel (8) zum Messen der Leitfähigkeit der Dialyseflüssigkeit beim Austreten aus dem Austauscher (1) umfaßt, dadurch gekennzeichnet, daß der Dialyseflussigkeitskreislauf (6) außerdem umfaßt,

– Mittel zum Bestimmen der Verzögerungszeit Tr der Dialyseflüssigkeit zwischen dem Eintreten in den Austauscher (1) und dem Austreten aus dem Austauscher (1), und

– Mittel zum Bestimmen des Leitfähigkeitswertes der Dialyseflüssigkeit im Gleichgewicht mit dem Plasma, für welchen die Leitfähigkeit der Dialyseflüssigkeit beim Austreten aus dem Austauscher (1) zu einem Zeitpunkt t gleich der Leitfähigkeit der Dialyseflüssigkeit beim Eintreten in den Austauscher zu einem Zeitpunkt tr = t – Tr ist.

10. Künstliche Niere nach Anspruch 9, dadurch gekennzeichnet, daß sie außerdem Mittel (7) für die Uberwachung der Leitfähigkeit der Dialyseflüssigkeit beim Eintreten in den Austauscher (1) umfaßt.

11. Künstliche Niere nach Anspruch 9 oder 10, dadurch gekennzeichnet, daß sie außerdem mittel (101) umfaßt zur Bestimmung des Natriämiewertes ausgehend von dem Leitfähigkeitswert der Dialyseflüssigkeit im Gleichgewicht mit dem Plasma.

12. Künstliche Niere nach den Ansprüchen 9 bis 11, dadurch gekennzeichnet, daß die Mittel für die Bestimmung der Verzögerungszeit Tr, als auch die Mittel zum Bestimmen der Leitfähigkeit der Dialyseflüssigkeit im Gleichgewicht mit dem Plasma und die Mittel zum Bestimmen des Natriämiewertes ausgehend von dem Wert der Leitfähigkeit der Dialyseflüssigkeit im Gleichgewicht durch einen Mikroprozessor ausgebildet sind.

## Claims

1. Method for determining the blood sodium level of a patient whose blood circulates through the first compartment (3) of an exchanger (1), separated by a semipermeable membrane (2) permitting the dialysis and ultrafiltration of the blood from a second compartment (4) through which a dialysis fluid circuit (6) passes, according to which the conductivity of the dialysis fluid in equilibrium with the plasma is determined and the relationship between this conductivity value and the sodium concentration in the plasma is then calculated, characterized in that the conductivity of the dialysis fluid in equilibrium with the plasma is determined by performing the following operations :

- the conductivity of the dialysis fluid at inflow to the exchanger is changed in a specified manner,
- the lag time TI of the dialysis fluid between inflow to and outflow from the exchanger is determined,
- the value of the conductivity for which the conductivity of the dialysis fluid at outflow from the exchanger at an instant t is equal to the conductivity of the dialysis fluid at inflow to the exchanger at an instant $t' = t - TI$ is determined, this value being the value of the conductivity of the dialysis fluid in equilibrium with the plasma.

2. Method according to Claim 1, characterized in that the conductivity of the dialysis fluid at inflow to the exchanger is changed in an increasing manner.

3. Method according to Claim 1, characterized in that the conductivity of the dialysis fluid at inflow to the exchanger is changed in a decreasing manner.

4. Method according to any one of the preceding claims, characterized in that the conductivity of the dialysis fluid at inflow to the exchanger is changed in a continuous manner.

5. Method according to any one of the preceding claims, characterized in that the determination of the lag time TI is performed in the following manner :

- at an instant t0, a sudden but brief change is produced in the conductivity of the dialysis fluid at inflow to the exchanger,
- the time elapsing between the instant t0 and the instant t1, at which a change in the conductivity of the dialysis fluid at outflow from the exchanger is detected by means of a conductimeter (8), is measured, this change being the response to the change in conductivity produced at inflow to the exchanger.

6. Method according to any one of Claims 1 to 4, characterized in that the determination of the lag time TI is performed in the following manner :

- a specified change in the conductivity of the dialysis fluid at inflow to the exchanger is produced, starting at an instant t0,
- the value for the conductivity of the dialysis fluid at outflow from the exchanger is measured,
- the equation for the curve 0 for the changes in the conductivity of the dialysis fluid at outflow from the exchanger with respect to time is determined,
- the instant t1, which is the abscissa of the point of change of slope of the curve O, is determined,
- the lag time TI, which is the time interval separating the instant t0 from the instant t1, is determined.

7. Method according to any one of the preceding claims, characterized in that the value employed for the conductivity of the dialysis fluid at inflow to the exchanger is the value measured by a conductimeter (7) placed upstream from the exchanger (1) with respect to the circulation of the dialysis fluid.

8. Method according to any one of Claims 1 to 6, characterized in that the value employed for the conductivity of the dialysis fluid at inflow to the exchanger (1) is a set value imposed by a monitoring device (9).

9. Artificial kidney comprising an exchanger (1) having at least two compartments (3, 4) separated by a semipermeable membrane (2) permitting the dialysis and ultrafiltration of the blood, the first compartment (3) being connected to an extracorporeal blood circuit (5) and the second compartment (4) being passed through by a dialysis fluid circuit (6) incorporating means (102) for changing the conductivity of the dialysis fluid at inflow to the exchanger (1) and means (8) for measuring the conductivity of the dialysis fluid at outflow from the exchanger (1), characterized in that the said dialysis fluid circuit (6) incorporates, in addition :

- means for determining the lag time TI of the dialysis fluid between inflow to and outflow from the exchanger (1),
- means for determining the conductivity value of the dialysis fluid in equilibrium with the plasma for which value the conductivity of the dialysis fluid at outflow from the exchanger (1) at an instant t is equal to the conductivity of the dialysis fluid at inflow to the exchanger at an instant $t' = t - TI$.

10. Artificial kidney according to Claim 9, characterized in that it incorporates, in addition, means (7) for measuring the conductivity of the dialysis fluid at inflow to the exchanger (1).

11. Artificial kidney according to Claim 9 and 10, characterized in that it incorporates, in addition, means (101) for determining the value of the blood sodium level from the value of the conductivity of the dialysis fluid in equilibrium with the plasma.

12. Artificial kidney according to any one of Claims 9 to 11, characterized in that the said means for determining the lag time Tl, as well as the said means for determining the conductivity of the dialysis fluid in equilibrium with the plasma and the said means for determining the value of the blood sodium level from the said conductivity value of the dialysis fluid at equilibrium, consist of a microprocessor.

FIGURE 1

FIGURE II

F IGURE III

FIGURE IV

EP 0 291 421 B1

EP 0 291 421 B1

F IGURE V

FIGURE VI

EP 0 291 421 B1